# EUROPEAN PATENT SPECIFICATION

(11) **EP 4 308 161 B1**
(45) Date of publication and mention of the grant of the patent: **30.04.2025**
(21) Application number: 21713940.1
(22) Date of filing: 19.03.2021
(51) Int. Cl.: A61K 41/00, A61P 27/02

(54) **OPHTHALMIC PREPARATIONS FOR PHOTO-CROSSLINKING REACTIONS**
OPHTHALMISCHE ZUBEREITUNGEN FÜR PHOTOVERNETZUNGSREAKTIONEN
PRÉPARATIONS OPHTALMIQUES POUR DES RÉACTIONS DE PHOTO-RÉTICULATION

(43) Date of publication of application: 24.01.2024
(73) Proprietor: Ligi Tecnologie Medicali S.r.l., 74100 Taranto (IT)
(72) Inventor: D'IPPOLITO, Giuseppe, 74121 Taranto (IT); MANGIULLI, Maurizio, 74121 Taranto (IT); LETTERA, Vincenzo, 80124 Napoli (IT)
(74) Representative: Bardehle Pagenberg Partnerschaft mbB Patentanwälte Rechtsanwälte
(86) International application number: PCT/EP2021/057147
(87) International publication number: WO 2022/194392

(56) References cited:
- WO-A1-2016/174688
- WO-A1-2018/156593
- WO-A2-2012/154627
- RU-C1- 2 646 452
- US-A1- 2018 050 104
- US-A1- 2018 236 261
- MORRISON PETER W J ET AL: "Enhancement in corneal permeability of riboflavin using calcium sequestering compounds", INTERNATIONAL JOURNAL OF PHARMACEUTICS, ELSEVIER, NL, vol. 472, no. 1, 10 June 2014 (2014-06-10), pages 56 - 64, XP029037407, ISSN: 0378-5173, DOI: 10.1016/J.IJPHARM.2014.06.007
- FILIPPELLO M ET AL: "Transepithelial corneal collagen crosslinking: bilateral study", JOURNAL CATARACT AND REFRACTIVE SURGERY, SURGERY, FAIRFAX, VA, US, vol. 38, no. 2, 1 February 2012 (2012-02-01), pages 283 - 291, XP002752018, ISSN: 0886-3350, [retrieved on 20111121], DOI: 10.1016/J.JCRS.2011.08.030
- LECCISOTTI ANTONIO ET AL: "Transepithelial corneal collagen cross-linking in keratoconus", JOURNAL OF REFRACTIVE SURGERY, SLACK INC, US, vol. 26, no. 12, 1 December 2010 (2010-12-01), pages 942 - 948, XP009168183, ISSN: 1081-597X, DOI: 10.3928/1081597X-20100212-09

## Description

### 1. Field of the invention

The present invention relates to aqueous solutions comprising chemical compounds that enhance photo-crosslinking reactions using photosensitizers. The enhancers related to the present invention increase speed and rate of the photo-crosslinking reaction and allows the photo crosslinking reaction to be activated also in hypoxia conditions, more specifically in the ophthalmic field, when the corneal tissue is soaked with an aqueous photo-sensitive solution and subsequently irradiated with, UV-A or visible light to treat ectatic corneal disorders.

### 2. Background

The cornea is a convex transparent tissue constituting the fundamental lens of the eye to focus the light onto the retina. The cornea derives its structural strength from corneal collagen. The strength of the intertwined collagen fibres is due to covalent crosslinks established between and within collagen fibres and between collagen fibres and glycoproteins of the stromal tissue.

The corneal structure may be subject to pathologic progressive viscous deformations, increasing over time under the permanent stress induced by the intraocular pressure. Such deformations are known as ectatic pathologies. A typical case of ectasia is the keratoconus. The ectatic pathologies show a localized reduction of the elastic module, induced by an intrastromal cross-linking deficiency of the stromal collagen fibres, which progressively generates a corneal pachymetry thinning due to the progressive slippage of the stromal fibres, induced by the permanent stress of the intraocular pressure, therefore, causing a localized ectasia of the corneal shape and a decrease of the quality of the vision.

Keratoconus affects about 1 in 2,000 people. It most commonly occurs in the late childhood to the early adulthood. Keratoconus is a genetic disease with a higher frequency amongst females and at times appears to be correlated to dysfunctions of endocrine glands (hypophysis and thyroid). It can affect both eyes in approximately 85% of cases and has an evolution that may vary from subject to subject. If the disease is neglected, in the worst scenario the apex of the keratoconus may ulcerate with possible perforation of the cornea, procuring pain, lacrimation and spasm of the eyelids and requiring corneal transplantation. The ectasia induces an alteration of the disposition of the corneal protein, causing micro-scars that may further distort the images and, in some cases, prevent the passage of light, thus giving rise to a troublesome dazzling feeling, above all at times of the day when the sun is low on the horizon.

In 1997, at the Ophthalmic Clinic of the Carl Gustaw Carus University of Dresden in Germany, a new safer and minimally invasive technique was developed, referred to as "corneal crosslinking", which using a riboflavin solution activated by a UV light (365-370 nm) enabled the reinforcement of the structure of the cornea affected by keratoconus increasing the crosslinking bonds between the fibres of the corneal collagen. Through this procedure the progression of the pathology was proved to be stopped or reduced. Several clinical studies have proven crosslinking procedure being able to slow down or halt ectasia progression, thus avoiding the need for transplantation of the cornea. It is by now widely and successfully used to halt the progression of corneal ectasia. The corneal crosslinking procedure is preferably carried out by applying a local corneal anaesthesia to debride the corneal epithelium (de-epithelization) up to a diameter of 8-9 mm. Consequently, the debrided cornea is soaked with the riboflavin solution followed by ultraviolet light (UV-A) irradiation.

Taking into account the photosynthesis phenomenon, scientists have tried to develop photosystems, based on photosensitizers, such as riboflavin, or alternatively, rose Bengal or methylene blue, which are capable of harvesting and converting light into chemical bounds through highly endothermic reactions.

When an electron of a photosensitising molecule with a singlet ground state is excited by absorption of UV radiation to a higher energy level, either an excited singlet state or an excited triplet state is activated. The molecule remains in the excited state of singlet for nanoseconds periods and then passes to the ground state by emitting a photon (fluorescence).

There are different phenomena that may occur after the absorption of a photon:
- the energy of the excited state can be dissipated non-radiatively in the form of heat;
- the energy of the excited state can be dissipated non-radiatively if the excited state decays into the lowest triplet state (intersystem crossing);
- the excited photosensitiser can collide with another molecule (quencher) to transfer energy in a second type of non-radiative process (quenching).

The main photochemical reactions based on flavins as photosensitizer involve intramolecular and intermolecular photoreduction, photoaddition and photodealkylation. These reactions have been reviewed in detail by Hemmerich (P. Hemmerich, B. Prijs, H. Erlenmeyer, Helv. Chim. Acta 1959, 42, 1604-1611) and Heelis (P. F. Heelis, R. F. Hartman, S. D. Rose, Chem. Soc. Rev. 1995, 24, 289; P. F. Heelis, Chem. Soc. Rev. 1982, 11, 15). The nature and magnitude of the photochemical reactions of flavins belongs to factors, such as solvent polarity, medium pH, buffer kind and composition like ionic strength, oxygen content, light intensities and wavelengths, and may proceed through the involvement of both "singlet-excited" and "triplet excited" states.

Riboflavin, also known as vitamin B, is poorly soluble in water, and therefore, riboflavin sodium phosphate, its freely water soluble derivative, is the preferred photosensitizing and photo-polymerizing molecule used to produce the ophthalmic solution to be delivered onto the patient's cornea during the therapeutic crosslinking treatment.

The patient's cornea, after being soaked with the photosensitizer, is irradiated with UV-A light. In presence of light, the photosensitizer is excited to a singlet state of higher energy, followed by non-radiative intersystem crossing to an excited triplet state. From this state, the photosensitizer can be involved in a photosensitized oxidation process following one of two following pathways:
- In the first pathway, "defined as Type-I", the photosensitizer transfers the energy to another molecule, present in the milieu, that rules as electron donor (also defined as sacrificial donor too) to the photosensitizer. The reaction produces photosensitizer free radical species generating oxidation products. This pathway occurs preferentially at low oxygen concentration.
- In the second pathway, "defined as Type-II", the photosensitizer transfers the energy to molecular oxygen in the ground state to generate the more reactive singlet molecular oxygen, the latter, that is reacting with a substrate like collagen, leading to the oxidation products.

In presence of UV-A light, riboflavin exhibits photosensitizing properties, reacting with a wide range of electron donating substrates, through mixed photochemical mechanisms that can involve or not the dissolved molecular oxygen.

As discussed above, in an oxygenated environment, a photosensitizer as riboflavin sodium phosphate or, alternatively, rose bengal or methylene blue, causes the formation of singlet oxygen, which produces additional crosslinked bonds on tissues as the stromal collagen fibres. The role of the oxygen in this process, especially in modulating photochemical kinetic mechanisms, is not completely understood.

On the other hand, at low oxygen concentrations, the photosensitizer, although with less effectiveness, may turn in a radical state, thus directly reacts with the collagen molecules producing additional crosslinked bonds on tissues.

From what is shortly described above, the collagen crosslinking is dependent from certain parameters like photosensitizer concentration, dissolved oxygen concentration and UV- A energy.

In the last decades, several different modified crosslinking protocols and techniques were established to increase the rate of the collagen crosslinking reactions to treat corneal ectasia. Different modulations of the UV-A energy irradiation were proposed, based on the Bunsen-Roscoe law of reciprocity (Mrochen M. Current status of accelerated corneal cross-linking. Indian J Ophthalmol. 2013 Aug;61(8):428-9). It was proposed delivering either a UV-A power of 9 mW/cm² for 10 minutes, or 18 mW/cm² for 5 minutes, or 30 mW/cm² for 3 minutes. Such modifications were designated as accelerated corneal crosslinking treatments, being the global UV-A amount of delivered energy the same, as per the standard crosslinking protocol. Applying any of these protocols, the treatment efficacy is limited by the fast oxygen depletion, (Hill J, Liu C, Deardorff P, Tavakol B, Eddington W, Thompson V, Gore D, Raizman M, Adler DC. Optimization of Oxygen Dynamics, UV-A Delivery, and Drug Formulation for Accelerated Epi-On Corneal Crosslinking. Curr Eye Res. 2020 Apr;45(4):450-458), thus further strategies has been developed to limit dissolved oxygen depletion during the radical reaction. The pulsed-light accelerated CXL is a procedure introduced to allow oxygen replenishment during the time lasting between UV-A pulses. Alternatively, extra oxygen availability into the corneal stroma may be achieved delivering onto the cornea highly oxygenated topical solutions, comprising lipid or oil-based fluids that are pre-oxygenated at high oxygen partial pressures, as described in WO 2013/148896 patent. US20120203161A1 discloses a riboflavin solution formulated into deuterated water to extend lifetime of UVA/Rf photo-generated intra-stromal singlet oxygen, in combination with UVA delivery profiles of pulsing, fractionation, and optionally auxiliary stromal/riboflavin hyper-oxygenation to accelerate protein cross-linking density rates in the ocular tissue.

However, for all reported photo-catalysed corneal crosslinking, the radical reduction mechanism is strictly dependent on molecular oxygen.

There are several cautions to be observed when using UV-A light with riboflavin sodium phosphate as photosensitizer for corneal crosslinking. Firstly, UV-A light can have cytotoxic effects on all living cells, and, specifically, on the corneal endothelial cells which can be destroyed by an excess of UV-A light or by reactive oxygen species. The cytotoxic hydrogen peroxide, which may be produced in poor oxygen environments, can kill, injure, or mutate healthy cells. Moreover, the rapid oxygen depletion, that occurs into the solution and into the corneal tissue during the photo catalysis, deeply limits the rate and the speed of crosslinking reaction.

Cherfan et al. (Cherfan D, Verter EE, Melki S, Gisel TE, Doyle FJ Jr, Scarcelli G, Yun SH, Redmond RW, Kochevar IE. Collagen cross-linking using rose Bengal and green light to increase corneal stiffness. Invest Ophthalmol Vis Sci. 2013 May 13;54(5):3426-33) have developed a collagen cross-linking technology that uses green light, a radiation source resulting less toxic for mammalian cells, to activate rose Bengal, however poverty of clinical data related to this procedure does not support the effectiveness of this technology. RU 2646452 C1 provides a composition containing riboflavin as photosentitizer for the treatment of corneal disorders such as keratoconus. The composition of D1 does not contain a secondary amine as enhancer but a primary amine, namely tris (hydroxymethyl) - methylamine as enhancer.

Hence, there is still a need of a more efficient ophthalmic photo-sensitizing solution to achieve a more efficient crosslinking reaction reducing the undesired toxic side effects, and consequently, producing a safer and more efficient treatment.

Consequently, a new formulation of a photosensitizing solution to maximize collagen crosslinking, both in presence or in absence of dissolved oxygen, to achieve a more efficient crosslinking reaction, increasing its rate and speed, and reducing undesired toxic side effects, is highly desirable.

### 3. Summary of the invention

The present invention relates to the finding of an enhancer boosting the photosensitizing activity of a photosensitizer, such as riboflavin sodium phosphate, or alternatively, rose bengal (4,5,6,7-tetrachloro-2',4',5',7'-tetraiodofluorescein) or methylene blue (also known as methylthioninium chloride or 3,7-Bis-(dimethylamino)-phenazathionium chloride), to maximize both the rate and the speed of the crosslinking reaction, for the treatment of corneal ectasia, to reduce treatment time and to improve surgical outcomes. The enhancer, object of the present invention, may be in particular a secondary amine such as ethylenediamine-N,N'-disuccinic acid (EDDS). For the reasons described below in detail, it acts as a catalytic enhancer, integrating the photosensitizing activity of a photosensitizer, such as riboflavin sodium phosphate, or, alternatively, rose Bengal or methylene blue, thus inducing a higher and faster rate of collagen crosslinking, in comparison to the state of the art.

### 4. Brief description of the invention

The problem is solved by the introduction of an enhancer increasing the photosensitizing activity of a photosensitizer, in particular a secondary amine, preferably ethylenediamine-N,N'-disuccinic acid (EDDS), belonging to the aminopolycarboxylic acids group. Such compound, e.g. EDDS, acts as enhancer of the photocatalysis reaction when added to a solution containing a photosensitizer such as riboflavin sodium phosphate or, alternatively, rosa Bengal or methylene blue.

EDDS, differently from other aminopolycarboxylic acids, such as EDTA, is naturally produced by a few microorganisms having a good degree of biodegradability (half-lives ranging from 2.5 days in a soil experiment to 4.6 days in an unacclimated Sturm test). Concerning biodegradability, the EDDS is an ideal additive to be used as electron donor to enhance the photocatalysis due to its rapid biodegradation.

The object of the present invention is the formulation of an aqueous solution containing the enhancer as described above added to a photosensitizer such as riboflavin sodium phosphate, or alternatively, rosa Bengal or methylene blue. Said aqueous solution can be prepared according to any known technique, to perform corneal crosslinking to treat more efficiently corneal ectasia pathologies.

The aqueous solution of the present invention can be prepared in the technical form of eye-drops, eyewashes, an ointment, and in any technical form allowing the delivery onto the cornea.

Hereinafter further embodiments of the present invention are described, without implying any limit to the present invention. Other aspects, advantages, and modifications within the scope of the invention will be apparent to those skilled in the art, to which the invention pertains.

The enhancer, in particular secondary amine, such as EDDS, increases the rate and the kinetics of the photo-induced reductive radical generation of the photosensitive solution, as below disclosed in experiment 1.

The inventors of the present invention have found that EDDS, a secondary amine, added to a solution containing riboflavin sodium phosphate as photosensitizer, or alternatively, rosa Bengal or methylene blue, acts as enhancer of the photo catalysis, deeply increasing the Type-I radical generation mechanism which is not efficient without the electron-donor activity of the EDDS.

Experiment 2, disclosed in the present invention as reported below, demonstrates that oxygen is not necessary to generate relevant quantities of reducing radicals, when a secondary amine, as the EDDS, is added to a solution containing riboflavin sodium phosphate as photosensitizer, or alternatively, rosa Bengal or methylene blue. In particular, the present invention demonstrates that the efficiency of radical generation in hypoxic conditions, when a secondary amine, as the EDDS, is added to a solution containing a photosensitizer as riboflavin sodium phosphate, or alternatively, rosa Bengal or methylene blue, is comparable to the efficiency of radical generation produced in an oxygenated microenvironment.

Therefore, the rate and the speed of the radical generation of a solution containing a secondary amine, as the EDDS, added as an enhancer to a solution containing a photosensitizer as the riboflavin sodium phosphate, or alternatively, the rosa Bengal or the methylene blue is almost independent by the oxygen diffusivity into the corneal tissue and by the oxygen consumption occurring during the UV-A light irradiation.

The present invention also discloses that the use of a secondary amine, as the EDDS, as enhancer of a solution containing a photosensitizer as riboflavin sodium phosphate or alternatively, rosa Bengal or methylene blue is more efficient compared to the use of tertiary amines, such as EDTA, as disclosed in the following experiment 3.

### 5. Description of the figure and tables

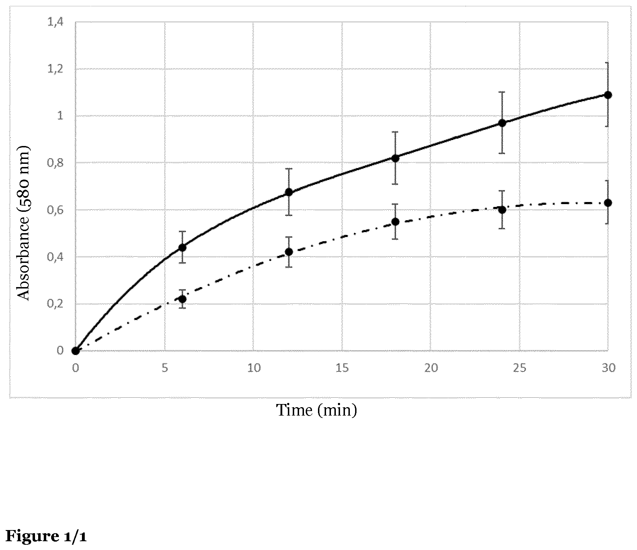

**Figure 1** - photo induced activity curves of the aqueous solution according to the invention (continuous line) containing 0.25% riboflavin sodium phosphate, 1.0% HPMC and 0.1% EDDS compared to a reference aqueous solution containing 0.25% riboflavin sodium phosphate, 1.0% HPMC without EDDS (dash and dot line).

The figure shows that the blue formazan absorbance at 580 nm, after 30 min of UV-A (365 nm) irradiation at 3.0 mW/cm², of the aqueous solution according to the invention, equal to 1.09, is almost 2 times higher of the blue formazan absorbance at 580 nm of the reference solution equal to 0.63.

**Table 1** - Blue formazan absorbance at 580 nm and blue formazan produced (µmol per ml of solution), after 30 min of UV-A (365 nm) irradiation at 9.0 mW/cm², of an aqueous solution according to the invention, containing 0.25% riboflavin sodium phosphate, 1.0% HPMC and 0.1% EDDS compared to a reference solution containing 0.25% riboflavin sodium phosphate, 1.0% HPMC without EDDS.

The table shows that the blue formazan produced (µmol per ml of solution), after 30 min of UV-A (365 nm) irradiation at 3.0 mW/cm², of the aqueous solution, equal to 0.88±0.13 µmol/ml, is 73% higher of the blue formazan produced (µmol per ml of the reference solution), being equal to 0.51±0.08 µmol/ml.

**Table 2** - Blue formazan absorbance at 580 nm and blue formazan produced µmol per ml of solution, after 30 min of UV-A (365 nm) irradiation at 9.0 mW/cm², of the aqueous solution of the embodiment 3, containing 0.25% riboflavin sodium phosphate, 1.0% HPMC and 0.1% EDDS compared to a reference solution containing 0.25% riboflavin sodium phosphate, 1.0% HPMC without EDDS in normal and low oxygen environmental conditions.

The table shows that the blue formazan produced µmol per ml of solution, in low oxygen environmental conditions, after 30 min of UV-A (365 nm) irradiation at 3.0 mW/cm², of the aqueous solution of the embodiment 3, equal to 0.75±0.11 µmol/ml, is higher compared to the blue formazan produced µmol per ml of the reference solution, equal to 0.20±0.03 µmol/ml. The activity ratio, between normal and low oxygen conditions, shows a minor decrement, equal to 15%, for the aqueous solution according to the invention, while it is 4 times higher, equal to 60% for the reference aqueous solution.

**Table 3** - Blue formazan produced µmol per ml of solution, after 30 min of UV-A (365 nm) irradiation at 9.0 mW/cm², of the aqueous solution of the embodiment 3, containing 0.25% riboflavin sodium phosphate, 1.0% HPMC and 0.1% EDDS compared to a reference solution containing 0.25% riboflavin sodium phosphate, 1.0% HPMC and 0.1% EDTA.

The table shows that the blue formazan produced µmol per ml of solution, after 30 min of UV-A (365 nm) irradiation at 3.0 mW/cm², of the aqueous solution of the embodiment 3, equal to 0.88±0.13 µmol/ml, is 22% higher of the blue formazan produced µmol per ml of the reference solution with 0.1% EDTA equal to 0.72±0.11 µmol/ml.

### 6. Detailed description of the invention

The invention is defined by the claims. Any subject-matter falling outside the scope of the claims is not part of the invention and provided for comparison or reference purposes only.

According to a first embodiment, an aqueous solution is disclosed comprising at least one enhancer, preferably selected from the group of the secondary amines, more preferably EDDS, and at least one photosensitizer, preferably riboflavin sodium phosphate, wherein the at least one photosensitizer is contained in a concentration between 0.01 and 0.5% w/v, preferably between 0.10 and 0.4% w/v, more preferably between 0.20 and 0.30% w/v, and wherein the at least one enhancer is contained in a concentration between 0.01 and 0.5% w/v, preferably 0.02 and 0.25% w/v, more preferably between 0.03 and 0.10% w/v.

According to a second embodiment, an aqueous solution is disclosed comprising at least one enhancer, preferably selected from the group of the secondary amines, more preferably EDDS, at least one photosensitizer, preferably riboflavin sodium phosphate and at least one polysaccharide and/or one polyethylene glycol (PEG) and/or one polyethylene oxide (PEO) and/or one poly(N-isopropylacrylamide) (PNIPAAm) and/or gelatine, preferably a polysaccharide, and in particular hydroxypropyl methylcellulose (HPMC), wherein the at least one photosensitizer is contained in a concentration between 0.01 and 0.5% w/v, preferably between 0.10 and 0.4% w/v, more preferably between 0.20 and 0.30% w/v, wherein the at least one enhancer is contained in a concentration between 0.01 and 0.5% w/v, preferably 0.02 and 0.25% w/v, more preferably between 0.03 and 0.10% w/v, and wherein the polysaccharide and/or one polyethylene glycol (PEG) and/or one polyethylene oxide (PEO) and/or one poly(N-isopropylacrylamide) (PNIPAAm) and/or gelatine is added in a concentration to achieve a viscosity of the aqueous solution between 3 and 10,000 cP (mPa ·s), preferably between 50 and 1000 cP (mPa · s), more preferably between 100 and 200 cP (mPa · s).

The inventors have found that a viscosity of the aqueous solution between 3 and 10,000 cP is required to provide, on the one hand, a stable film on the cornea while, on the other hand, still allowing for a homogeneous distribution onto the corneal shape after application.

The viscosity of the aqueous solutions of the second embodiment is optimized to homogeneously spread the aqueous solution on the surface of the cornea and to contextually grant a fast penetration of the aqueous solution into the corneal tissue. A lower viscosity, below the above described limits, would induce a too short break-up time of the aqueous solution film onto the corneal surface with less efficient damage protection against damages caused by an UV-A light direct exposition of the corneal tissue; conversely an higher viscosity, above the above described limits, would negatively affect the speed of penetration of the aqueous solution into the corneal structure, thus increasing the total treatment time and reducing the capability to generate radicals.

According to a third embodiment an aqueous solution is disclosed comprising at least one enhancer, preferably selected from the group of the secondary amines, more preferably the EDDS, at least one photosensitizer, preferably the or riboflavin sodium phosphate, at least one polysaccharide, preferably the HPMC and at least one buffering agent, preferably sodium tetra borate /boric acid (i.e. a borate-boric acid buffer system), wherein the at least one photosensitizer is contained in a concentration between 0.01 and 0.5% w/v, preferably between 0.10 and 0.4% w/v, more preferably between 0.20 and 0.30% w/v, wherein the at least one enhancer is contained in a concentration between 0.01 and 0.5% w/v, preferably 0.02 and 0.25% w/v, more preferably between 0.03 and 0.10% w/v, wherein the polysaccharide is added in a concentration to achieve a viscosity of the aqueous solution between 3 and 10,000 cP, preferably between 50 and 1000 cP, more preferably between 100 and 200 cP, and wherein the buffering agent is added in a concentration to achieve an osmolality between 240 and 300 mOsm/Kg, preferably a osmolality between 250 and 290 mOsm/Kg, more preferably a osmolality between 260 and 280 mOsm/Kg.

The osmolality of the aqueous solutions of the third embodiment is optimized to achieve mild hypotonic conditions to facilitate the replacement of the water that evaporates from the corneal stroma during the crosslinking treatment. A lower osmolality, below the above-described limits, would induce excessive water replenishment causing stromal swelling and reducing the capability of crosslinking bonds activation; conversely an higher osmolality, above the above described limits, would induce stromal dehydration, causing stromal shrinkage and risk of endothelium damages.

The following experiment 1 demonstrates that the addition of a secondary amine, in particular EDDS, to an aqueous solution containing a photosensitizer such as riboflavin sodium phosphate or alternatively, rosa Bengal or methylene blue, enhances the photo catalysis, relevantly increasing rate and speed of the radical production, in comparison to a reference photosensitive solution without the addition of the secondary amine, as the EDDS.

The following experiment 2 demonstrates that the rate and the speed of the radical generation of an aqueous solution containing a photosensitizer as riboflavin sodium phosphate or alternatively, rosa Bengal or methylene blue, added with a secondary amine, such as EDDS, is quasi-independent by the oxygen presence during the UV-A light irradiation, differently from a reference photosensitive solution without the addition of a secondary ammine, such as EDDS.

The following experiment 3 demonstrates that the addition of a secondary amine, such as EDDS, to a solution containing a photosensitizer as the riboflavin sodium phosphate or alternatively, rosa bengal or methylene blue, provides a more efficient enhancement of the rate and speed of the radical production, in comparison to a reference solution added with a tertiary amine, as the EDTA.

**Experiment 1 - Determination of the ability to generate reducing radicals of the aqueous solution of the embodiment 3, containing 0.25% of riboflavin sodium phosphate, 0.1% of EDDS and 1.0% of HPMC compared to a reference solution containing 0.25% of riboflavin sodium phosphate and 1.0% of HPMC.**

The photo-chemical production of radicals of the two aqueous solutions was determined monitoring the reduction of the nitro-blue tetrazolium (NBT) which was converted in blue formazan. The rate of the produced radicals is directly proportional to the measured amount of blue formazan mols. The blue formazan generation causes a colour change from yellow to blue detectable with a spectrophotometer at 580 nm wavelength.

An assay of 100 µl of the two aqueous solutions to be tested was mixed with 9 µl of NBT aqueous solution (10mg/ml), put in a well of a microtiter plate (48-well Microtiterplate - Nunclon deltasurface; ThermoScientific) and, subsequently irradiated for 30 min at 3.0 mW/cmq with the CCL-VARIO UV-A (365 nm) lamp produced by M-Lase, using a 10.0 mm aperture at 5.0 cm distance.

The UV-A irradiation, for both solutions, was conducted in a normal oxygenated environment (room air, 21% oxygen). The blue formazan absorbance at 580 nm was measured, using the spectrophotometer UV-1600PC VWR, after having been diluted the samples of the two aqueous solutions, in a1:10 ratio, with 900 µl of water in 1ml cuvette. The measured absorbance values were then divided by the molar extinction coefficient of the blue formazan at 580 nm (12,300 M⁻¹ cm⁻¹) to determine the reduced NBT concentration.

The spectrophotometric measurements of the aqueous solution of the embodiment 3, containing 0.25% of riboflavin sodium phosphate, 0.1% of EDDS and 1.0% of HPMC having a dynamic viscosity of 137 cP at 25 °C vs. the spectrophotometric measurements of the reference solution containing 0.25% riboflavin sodium phosphate and 1.0% of HPMC having a dynamic viscosity of 143 cP at 25 °C are reported in figure 1.

Figure 1 shows that the blue formazan absorbance at 580 nm, after 30 min of UV-A (365 nm) irradiation at 3.0 mW/cmq, of the aqueous solution of the embodiment 3, equal to 1.09, is almost 2 times higher of the blue formazan absorbance at 580 nm of the reference solution equal to 0.63).

The data reported in the Table 1 show that the blue formazan produced µmol per ml of solution, after 30 min of UV-A (365 nm) irradiation at 3.0 mW/cmq, of the aqueous solution of the embodiment 3, equal to 0,88±0,13 µmol/ ml, is 73% higher of the formazan produced µmol per ml of the reference solution equal to 0.51±0.08 µmol/ ml).

Experiment 1 shows that both rate and speed of the radical production of the aqueous solution of the embodiment 3 are relevantly higher in comparison with the reference solution.

**Experiment 2** - **Determination of the ability to generate reducing radicals, in hypoxic conditions, of the aqueous solution of the embodiment 3, containing 0.25% of riboflavin sodium phosphate, 0.1% of EDDS and 1.0% of HPMC compared to a reference solution containing 0.25% of riboflavin sodium phosphate and 1.0% of HPMC.**

The photo-chemical production of radicals of the two aqueous solutions was determined monitoring the reduction of the nitro-blue tetrazolium (NBT) which was converted in blue formazan. The rate of the produced radicals is directly proportional to the measured amount of blue formazan mols. The blue formazan generation causes a colour change from yellow to blue detectable with a spectrophotometer at 580 nm wavelength.

An assay of 100 µl of the two aqueous solutions to be tested was mixed with 9 µl of NBT aqueous solution (10mg/ml), put in a well of a microtiter plate (48-well Microtiterplate - Nunclon deltasurface; ThermoScientific) and, consequently irradiated for 30 min at 3.0 mW/cmq with the CCL-VARIO UV-A (365 nm) lamp produced by M-Lase, using a 10.0 mm aperture at 5.0 cm distance.

The UV-A irradiation, for both solutions, was conducted in a low-level oxygenated environment (<0.1% oxygen, nitrogen atmosphere). Nitrogen bubbling was also carried out, for 10 minutes, into the two aqueous solutions to strip dissolved oxygen from the aqueous solutions before the UV-A irradiation. The blue formazan absorbance at 580 nm was measured, using the spectrophotometer UV-1600PC VWR, after having been diluted the samples of the two aqueous solutions, in a1:10 ratio, with 900 µl of water in 1ml cuvette. The measured absorbance values were then divided by the molar extinction coefficient of the blue formazan at 580 nm (12,300 M⁻¹ cm⁻¹) to determine the reduced NBT concentration.

The spectrophotometric measurements of the aqueous solution of the embodiment 3, containing 0.25% of riboflavin sodium phosphate, 0.1% of EDDS and 1.0% of HPMC having a dynamic viscosity of 137 cP at 25 °C vs. the reference solution containing 0.25% of riboflavin sodium phosphate and 1.0% of HPMC having a dynamic viscosity of 143 cP at 25 °C are reported in the table 2, which compares the blue formazan absorbance at 580nm and the blue formazan production of the two aqueous solutions, both in normal and in low oxygen environments.

Table 2 shows that the blue formazan produced µmol per ml of solution, in low oxygen environmental conditions, after 30 min of UV-A (365 nm) irradiation at 3.0 mW/cmq, of the aqueous solution of the embodiment 3, equal to 0.75±0.11 µmol/ml, is sensitively higher of the blue formazan produced µmol per ml of the reference solution, equal to 0.20±0.03 µmol/ml.

Table 2 shows that the blue formazan production of the reference solution, in low oxygen environments, is relevantly reduced of 60% in comparison with the blue formazan production in normal oxygen environments, whereas the blue formazan production of the aqueous solution of the embodiment 3, in low oxygen environments, is only reduced of 15% in comparison with the blue formazan production in normal oxygen environments.

Experiment 2 shows that both rate and speed of the radical production of the aqueous solution of the embodiment 3 are increased in comparison with the reference solution, being, rate and speed of the radical production, in low level conditions, quasi- identical to rate and speed of the radical production, in normal level conditions, for the aqueous solution of embodiment 3.

**Experiment 3 - Determination of the ability to generate reducing radicals of the aqueous solution of the embodiment 3, containing 0.25% riboflavin sodium phosphate, 0.1% of EDDS and 1.0% of HPMC compared to a reference solution containing 0.25% of riboflavin sodium phosphate, 0.1% of EDTA and 1.0% of HPMC.**

The photo-chemical production of radicals of the two aqueous solutions was determined monitoring the reduction of the nitro-blue tetrazolium (NBT) which was converted in blue formazan. The rate of the produced radicals is directly proportional to the measured amount of blue formazan mols. The blue formazan generation causes a colour change from yellow to blue detectable with a spectrophotometer at 580 nm wavelength.

An assay of 100 µl of the two aqueous solutions to be tested was mixed with 9 µl of NBT aqueous solution (10mg/ml), put in a well of a microtiter plate (48-well Microtiterplate - Nunclon deltasurface; ThermoScientific) and, consequently irradiated for 30 min at 3.0 mW/cmq with the CCL-VARIO UV-A (365 nm) lamp produced by M-Lase, using a 10.0 mm aperture at 5.0 cm distance.

The UV-A irradiation, for both solutions, was conducted in a normal oxygenated environment (room air, 21% oxygen). The blue formazan absorbance at 580 nm was measured, using the spectrophotometer UV-1600PC VWR, after having been diluted the samples of the two aqueous solutions, in a1:10 ratio, with 900 µl of water in 1ml cuvette. The measured absorbance values were then divided by the molar extinction coefficient of the blue formazan at 580 nm (12,300 M⁻¹ cm⁻¹) to determine the reduced NBT concentration.

The blue formazan production in µmol per ml of solution of the embodiment 3, containing 0.25% of riboflavin sodium phosphate, 0.1% of EDDS and 1.0% of HPMC vs. the blue formazan production in µmol per ml of the reference solution containing 0.25% of riboflavin sodium phosphate, o,1% of EDTA and 1.0% of HPMC are reported in figure 3.

Figure 3 shows that the formazan produced µmol per ml of solution, after 30 min of UV-A (365 nm) irradiation at 3.0 mW/cmq, of the aqueous solution of the embodiment 3, equal to 0,88±0,13 µmol/ml, is 22% higher of the formazan produced µmol per ml of the reference solution with 0.1% EDTA equal to 0.72±0.11 µmol/ml.

Experiment 3 shows that the radical production of the aqueous solution of the embodiment 3, with 0.1% EDDS is more efficient in comparison with the reference solution with 0.1% EDTA.

In conclusion the above conducted experiments confirm that the addition of a secondary amine as the EDDS to an aqueous solution containing a photosensitizer as the riboflavin sodium phosphate, relevantly increases rate and speed of the crosslinking reaction and that the addition of a secondary amine as the EDDS is more efficient of the addition of a tertiary amine as the EDTA.

### TABLES

**Table 1**

| | **Absorbance at 580 nm after 30 min of UV-A (365 nm) irradiation at 3.0 mW/cm²** | **Produced formazan (µmol/ml)** | **Produced Formazan (%) normalized with respect to reference solution** |
|---|---|---|---|
| **Reference solution** | 0.63±0.09 | 0.51±0.08 | 100 |
| **Embodiment 3 solution** | 1.09±0.16 | 0.88±0.13 | 173% |

**Table 2**

| | **Produced formazan (µmol/ml) in normal oxygen conditions** | **Produced formazan (µmol/ml) in low-oxygen conditions** | **Decrement activity ratio between normal and low oxygen conditions (%)** |
|---|---|---|---|
| **Reference solution** | 0.51±0.08 | 0.20±0.03 | 60% |
| **Embodiment 3 solution** | 0.88±0.13 | 0.75±0.11 | 15% |

**Table 3**

| | **Produced formazan (µmol/ml)** | **Produced Formazan (%) normalized with respect to reference solution + 0.1% EDTA** |
|---|---|---|
| **Reference solution + 0.1% EDTA** | 0.72±0.11 | 100 |
| **Embodiment 3 solution** | 0.88±0.13 | 122% |

## Claims

1. An aqueous solution comprising:
(i) at least one photosensitizer selected from the group consisting of riboflavin sodium phosphate, rose bengal and methylene blue, and
(ii) at least one enhancer increasing the photosensitizing activity of a photosensitizer,
(iii) at least a viscous agent controlling the viscosity of the aqueous solution;
wherein the at least one photosensitizer is contained in a concentration between 0.01 and 0.5% w/v,
wherein the at least one enhancer is an electron donor, belongs to the group of secondary amines, and is contained in a concentration between 0.01 and 0.5% w/v,
and wherein the aqueous solution has a viscosity of between 3 and 10,000 mPa · s.

2. The aqueous solution according to claim 1, wherein the at least one enhancer is ethylenediamine-N,N'-disuccinic acid.

3. The aqueous solution, according to any one of claims 1 to 2, wherein the viscous agent is selected from the group consisting of a polysaccharide, a polyethylene glycol, a polyethylene oxide, and a poly(N-isopropylacrylamide).

4. The aqueous solution according to claim 3, wherein the polysaccharide is a cellulose derivative, preferably selected from methylcellulose, hydroxypropyl methylcellulose, hydroxy ethyl methyl cellulose, carboxy-methyl-cellulose, further preferably hydroxypropyl methylcellulose.

5. The aqueous solution according to any one of claims 1 to 4, wherein the at least one photosensitizer is contained in a concentration between 0.10 and 0.4% w/v, preferably between 0.20 and 0.30% w/v.

6. The aqueous solution according to any one of claims 1 to 5, wherein the at least one enhancer is contained in a concentration between 0.02 and 0.25% w/v, preferably between 0.03 and 0.10% w/v.

7. The aqueous solution according to any one of claims 1 to 6, wherein the aqueous solution has a viscosity of between 50 and 1000 mPa . s, preferably between 100 and 200 mPa . s.

8. The aqueous solution according to any one of claims from 1 to 7, further comprising at least one buffering agent.

9. The aqueous solution according to claim 8, having an osmolality between 240 and 300 mOsm/Kg, preferably a osmolality between 250 and 290 mOsm/Kg, more preferably a osmolality between 260 and 280 mOsm/Kg.

10. The aqueous solution according to claim 8 or 9, wherein the buffering agent comprises or consists of sodium tetraborate and boric acid.

11. The aqueous solution according to any one of claims from 1 to 10, comprising at least one polysaccharide, and at least one buffering agent, wherein the aqueous solution has a viscosity of between 3 and 10,000 mPa. s, preferably between 50 and 1000 mPa . s, more preferably between 100 and 200 mPa · s, and a osmolality of between 240 and 300 mOsm/Kg, preferably a osmolality between 250 and 290 mOsm/Kg, more preferably a osmolality between 260 and 280 mOsm/Kg.

12. The aqueous solution according to claim 11, wherein the polysaccharide is hydroxypropyl methylcellulose and the buffering agent comprises sodium tetraborate and boric acid.

13. An aqueous solution according to any one of the preceding claims for use in the treatment of corneal disorders, in particular corneal ectatic disorders.

14. The aqueous solution for use according to claim 13, wherein the aqueous solution is applied to the cornea of a patient and irradiated with UV-A or visible light for inducing a cross-linking reaction.

## Patentansprüche

1. Wässrige Lösung, umfassend:
(i) mindestens einen Photosensibilisator, ausgewählt aus der Gruppe, bestehend aus Riboflavinnatriumphosphat, Bengalrosa und Methylenblau, und
(ii) mindestens einen Verstärker, der die Photosensibilisierungsaktivität eines Photosensibilisators erhöht,
(iii) mindestens ein viskoses Mittel, das die Viskosität der wässrigen Lösung steuert;
wobei der mindestens eine Photosensibilisator in einer Konzentration zwischen 0,01 und 0,5 % w/v enthalten ist,
wobei der mindestens eine Verstärker ein Elektronendonor ist, zur Gruppe der sekundären Amine gehört und in einer Konzentration zwischen 0,01 und 0,5 % w/v enthalten ist,
und wobei die wässrige Lösung eine Viskosität zwischen 3 und 10.000 mPa · s aufweist.

2. Wässrige Lösung nach Anspruch 1, wobei der mindestens eine Verstärker Ethylendiamin-N,N'-dibernsteinsäure ist.

3. Wässrige Lösung nach einem der Ansprüche 1 bis 2, wobei das viskose Mittel ausgewählt ist aus der Gruppe, bestehend aus einem Polysaccharid, einem Polyethylenglykol, einem Polyethylenoxid und einem Poly(N-isopropylacrylamid).

4. Wässrige Lösung nach Anspruch 3, wobei das Polysaccharid ein Cellulosederivat ist, vorzugsweise ausgewählt aus Methylcellulose, Hydroxypropylmethylcellulose, Hydroxyethylmethylcellulose, Carboxymethylcellulose, weiter vorzugsweise Hydroxypropylmethylcellulose.

5. Wässrige Lösung nach einem der Ansprüche 1 bis 4, wobei der mindestens eine Photosensibilisator in einer Konzentration zwischen 0,10 und 0,4 % w/v, vorzugsweise zwischen 0,20 und 0,30 % w/v enthalten ist.

6. Wässrige Lösung nach einem der Ansprüche 1 bis 5, wobei der mindestens eine Verstärker in einer Konzentration zwischen 0,02 und 0,25 % w/v, vorzugsweise zwischen 0,03 und 0,10 % w/v enthalten ist.

7. Wässrige Lösung nach einem der Ansprüche 1 bis 6, wobei die wässrige Lösung eine Viskosität zwischen 50 und 1000 mPa · s, vorzugsweise zwischen 100 und 200 mPa · s aufweist.

8. Wässrige Lösung nach einem der Ansprüche 1 bis 7, weiter umfassend mindestens ein Puffermittel.

9. Wässrige Lösung nach Anspruch 8, die eine Osmolalität zwischen 240 und 300 mOsm/kg, vorzugsweise eine Osmolalität zwischen 250 und 290 mOsm/kg, mehr bevorzugt eine Osmolalität zwischen 260 und 280 mOsm/kg aufweist.

10. Wässrige Lösung nach Anspruch 8 oder 9, wobei das Puffermittel Natriumtetraborat und Borsäure umfasst oder daraus besteht.

11. Wässrige Lösung nach einem der Ansprüche 1 bis 10, umfassend mindestens ein Polysaccharid und mindestens ein Puffermittel, wobei die wässrige Lösung eine Viskosität zwischen 3 und 10.000 mPa · s, vorzugsweise zwischen 50 und 1000 mPa · s, mehr bevorzugt zwischen 100 und 200 mPa · s, und eine Osmolalität zwischen 240 und 300 mOsm/kg, vorzugsweise eine Osmolalität zwischen 250 und 290 mOsm/kg, mehr bevorzugt eine Osmolalität zwischen 260 und 280 mOsm/kg aufweist.

12. Wässrige Lösung nach Anspruch 11, wobei das Polysaccharid Hydroxypropylmethylcellulose ist und das Puffermittel Natriumtetraborat und Borsäure umfasst.

13. Wässrige Lösung nach einem der vorstehenden Ansprüche zur Verwendung bei der Behandlung von Hornhauterkrankungen, insbesondere ektatische Hornhauterkrankungen.

14. Wässrige Lösung zur Verwendung nach Anspruch 13, wobei die wässrige Lösung auf die Hornhaut eines Patienten aufgetragen und mit UV-A oder sichtbarem Licht bestrahlt wird, um eine Vernetzungsreaktion zu induzieren.

## Revendications

1. Solution aqueuse comprenant :
(i) au moins un photosensibilisateur choisi dans le groupe constitué par le phosphate sodique de riboflavine, le rose bengale et le bleu de méthylène, et
(ii) au moins un renforçateur augmentant l'activité photosensibilisante d'un photosensibilisateur,
(iii) au moins un agent visqueux ajustant la viscosité de la solution aqueuse ;
dans laquelle l'au moins un photosensibilisateur est présent à une concentration entre 0,01 et 0,5 % p/v,
et dans laquelle l'au moins un renforçateur est un donneur d'électrons, appartient au groupe des amines secondaires et est présent à une concentration entre 0,01 et 0,5 % p/v,
et dans laquelle la solution aqueuse ayant une viscosité entre 3 et 10 000 mPa·s.

2. Solution aqueuse selon la revendication 1, dans laquelle l'au moins un renforçateur est l'acide éthylènediamine-N,N'-disuccinique.

3. Solution aqueuse selon l'une des revendications 1 à 2, dans laquelle l'agent visqueux est choisi dans le groupe constitué par un polysaccharide, un polyéthylèneglycol, un poly(oxyde d'éthylène) et un poly(N-isopropylacrylamide).

4. Solution aqueuse selon la revendication 3, dans laquelle le polysaccharide est un dérivé de la cellulose, de préférence choisi parmi la méthylcellulose, l'hydroxypropylméthylcellulose,
l'hydroxyéthylméthylcellulose, la carboxy-méthylcellulose, plus préférentiellement l'hydroxypropylméthylcellulose.

5. Solution aqueuse selon l'une des revendications 1 à 4, dans laquelle l'au moins un photosensibilisateur est présent à une concentration entre 0,10 et 0,4 % p/v, de préférence entre 0,20 et 0,30 % p/v.

6. Solution aqueuse selon l'une des revendications 1 à 5, dans laquelle l'au moins un renforçateur est présent à une concentration entre 0,02 et 0,25 % p/v, de préférence entre 0,03 et 0,10 % p/v.

7. Solution aqueuse selon l'une des revendications 1 à 6, dans laquelle la solution aqueuse a une viscosité entre 50 et 1 000 mPa·s, de préférence entre 100 et 200 mPa·s.

8. Solution aqueuse selon l'une des revendications 1 à 7, comprenant en outre au moins un agent tampon.

9. Solution aqueuse selon la revendication 8, ayant une osmolalité entre 240 et 300 mOsm/kg, de préférence une osmolalité entre 250 et 290 mOsm/kg, plus préférentiellement une osmolalité entre 260 et 280 mOsm/kg.

10. Solution aqueuse selon la revendication 8 ou 9, dans laquelle l'agent tampon comprend du tétraborate de sodium et de l'acide borique ou en est constitué.

11. Solution aqueuse selon l'une des revendications 1 à 10, comprenant au moins un polysaccharide et au moins un agent tampon, la solution aqueuse ayant une viscosité entre 3 et 10 000 mPa·s, de préférence entre 50 et 1 000 mPa·s, plus préférentiellement entre 100 et 200 mPa·s, et une osmolalité entre 240 et 300 mOsm/kg, de préférence une osmolalité entre 250 et 290 mOsm/kg, plus préférentiellement une osmolalité entre 260 et 280 mOsm/kg.

12. Solution aqueuse selon la revendication 11, dans laquelle le polysaccharide est l'hydroxypropylméthylcellulose et l'agent tampon comprend du tétraborate de sodium et de l'acide borique.

13. Solution aqueuse selon l'une des revendications précédentes, pour une utilisation dans le traitement de troubles de la cornée, en particulier de troubles ectasiques de la cornée.

14. Solution aqueuse pour une utilisation selon la revendication 13, dans laquelle la solution aqueuse est appliquée sur la cornée d'un patient et irradiée avec une lumière UV-A ou visible pour induire une réaction de réticulation.
